Europäisches Patentamt

European Patent Office

Office européen des brevets

(11) Numéro de publication : **0 443 891 B1**

# (12) FASCICULE DE BREVET EUROPEEN

(45) Date de publication du fascicule du brevet :
**15.09.93 Bulletin 93/37**

(51) Int. Cl.⁵ : **A61K 31/20**

(21) Numéro de dépôt : **91400163.1**

(22) Date de dépôt : **25.01.91**

(54) **Utilisation de la N-myristoyl-(S)-phénylalanine pour l'obtention de médicaments destinés au traitement des maladies faisant intervenir la myristoylation.**

(30) Priorité : **25.01.90 FR 9000834**

(43) Date de publication de la demande :
**28.08.91 Bulletin 91/35**

(45) Mention de la délivrance du brevet :
**15.09.93 Bulletin 93/37**

(84) Etats contractants désignés :
**AT BE CH DE DK ES FR GB GR IT LI LU NL SE**

(56) Documents cités :
CHEMICAL ABSTRACTS, vol. 109, no. 25, 19 décembre 1988, page 919, abrégé no. 231563w, Columbus, Ohio, US;

(56) Documents cités :
PROC. NATL. ACAD. SCI. USA, vol. 86, novembre 1989, pages 8655-8659; M.L. BRYANT et al.: "Replication of human immunodeficiency virus 1 and Moloney murine leukemia virus is inhibited by different heteroatom-containing analogs of myristic acid"
CHEMICAL ABSTRACTS, vol. 76, no. 20, 15 mai 1972, pages 319-320, abrégé no. 117436g, Columbus, Ohio, US; & JP-A-71 14 357 (AJINOMOTO CO., INC.) 17-04-1971

(73) Titulaire : **ADIR ET COMPAGNIE**
**1 rue Carle Hébert**
**F-92415 Courbevoie Cédex (FR)**

(72) Inventeur : **Vincent, Michel**
**8 allée du Prunier Hardy**
**F-92220 Bagneux (FR)**
Inventeur : **Remond, Georges**
**9 avenue des Etats-Unis**
**F-78000 Versailles (FR)**
Inventeur : **Hervé, Yolande**
**16 rue Eichenberger**
**F-92800 Puteaux (FR)**
Inventeur : **Boutin, Jean-Albert**
**22 esplanade des Courtieux**
**F-92150 Suresnes (FR)**

EP 0 443 891 B1

## Description

La présente invention a pour objet l'utilisation de la N-myristoyl-(S)-phénylalanine pour l'obtention d'une composition pharmaceutique utilisable en tant qu'inhibiteur de la N-myristoyl transférase (NMT).

Il est connu de l'art antérieur, que le groupement amino N-terminal des protéines est bloqué par des groupements acétyle, pyroglutamyle et formyle. Or, SHOJI et Coll. ont mis en évidence que l'acide myristique était lié par une liaison covalente au groupement N-terminal des sous-unités catalytiques de la protéine kinase AMP cyclique dépendante (Proc. Natl. Acad. Sci. USA, (1982), 79, 6123-6131).

L'existence de ce groupement myristoyl terminal a depuis lors été montré dans diverses autres protéines comme la calcineurine B (AITKEN et Coll, Febs Letters, (1982), 150, n° 2, 314-318) ou la tyrosine protéine kinase (TPK) (BUSS et SEFTON, J. Virol, (1985),53, 7-12).

Dans le domaine des oncogènes également, BISHOP a mis en évidence qu'une protéine transformante subissait une myristoylation lors de la maturation. Il a d'ailleurs été montré depuis que cette étape de maturation passant par une myristoylation était essentielle au pouvoir transformant de cette protéine (KAMPS, BUSS et SEFTON, Proc. Natl. Acad. Sci. USA, (1985), 82, 4625-4628). Ce concept a dès lors été généralisé à de nombreuses autres protéines transformantes d'origine virale (RHEE et HUNTER, J. Virol., (1987), 61, 1045-1053). Cette maturation est catalysée par une enzyme appelée N-myristoyltransférase mise en évidence par TOWLER et GLASER (Proc. Natl. Acad. Sci. USA, (1986), 83, 2812-2816).

Or, la NMT ne reconnait pratiquement comme cosubstrat que l'acide myristique d'une part et d'autre part, comme substrat, les protéines comportant une glycine comme dernier acide aminé du côté N-terminal, avec participation de la séquence peptidique jouxtant cette glycine (participation de 7 amino-acides).

Ainsi, la myristoylation du résidu glycine N-terminal de certaines protéines joue un rôle très important sur certains mécanismes intervenant dans la transformation des cellules et le contrôle de leur prolifération. Il a d'ailleurs été montré par SHOJI et Coll. (brevets japonais JP 63-146851, JP 62-255810 et JP 62-126384) que la myristoylglycine ou des dérivés oligopeptidiques possédait un effet inhibiteur contre la transformation ou la prolifération cellulaire ou la multiplication de rétrovirus.

L'invention consiste plus particulièrement en l'utilisation de la N-myristoyl-(S)-phénylalanine pour l'obtention d'une composition pharmaceutique utilisable comme inhibiteur de la myristoylation de protéines telles que gag par le biais de l'enzyme responsable de cette myristoylation, c'est-à-dire de la N-myristoyltransférase.

L'inhibition de l'activité de cette enzyme n'avait jusqu'alors jamais été mise en évidence par ce composé. De plus, l'utilisation de la N-myristoyl-(S)-phénylalanine en tant qu'inhibiteur de NMT conduit à une inhibition de l'activité de cette enzyme nettement supérieure à celle de composés décrits dans l'art antérieur comme inhibiteurs de la prolifération de cellules cancéreuses et de rétrovirus.

En effet, une étude approfondie de l'influence exercée par la N-myristoyl-(S)-phénylalanine sur la prolifération et la transformation cellulaire a été réalisée à partir de cellules cancéreuses d'origine murine (L 1210). Après extraction de l'enzyme de ce milieu biologique et mesure de son activité, il apparaît que l'addition de N-myristoyl-(S)-phénylalanine inhibe fortement son activité.

De plus, ce composé présente une activité cytotoxique sur des cellules cancéreuses en culture telles que L 1210 (d'origine murine) ou HL 60 (d'origine humaine). Cette cytotoxicité s'est révélée être nettement supérieure à celle due à la N-myristoylglycine sur ces cellules.

Ainsi, cette inhibition d'activité est d'autant plus intéressante que cette enzyme joue un rôle prépondérant, en particulier dans la maturation soit de protéines transformantes impliquées dans certains cancers, soit de protéines elles-mêmes impliquées dans la maturation de rétrovirus.

La N-myristoyl-(S)-phénylalanine a donc potentiellement des applications dans le traitement du cancer et/ou de maladies à rétrovirus comme le SIDA.

La présente invention a également pour objet l'utilisation de la N-myristoyl -(S)-phénylalanine ou d'un de ses sels d'addition à une base pharmaceutiquement acceptable, sous la forme d'une composition pharmaceutique seule ou en combinaison avec un ou plusieurs excipients ou véhicules inertes non toxiques.

Parmi les compositions pharmaceutiques selon l'invention, on pourra citer celles qui conviennent pour l'administration orale, parentérale, nasale, les comprimés simples ou dragéifiés, les comprimés sublinguaux, les sachets, les gélules, les suppositoires, etc...

La posologie varie selon l'âge et le poids du patient, la nature et la sévérité de l'affection ainsi que la voie d'administration. Celle-ci peut être orale, nasale, rectale ou parentérale. D'une manière générale, la posologie unitaire s'échelonne entre 0,1 et 100 mg pour un traitement en 1 à 3 prises par 24 heures.

Les exemples suivants illustrent l'invention.

**Exemple 1 : Synthèse de la N-myristoyl-(S)-phénylalanine**

**Stade A** : N-myristoyl-(S)-phénylalaninate de terbutyle

En utilisant la technique décrite par VAUGHAN J.R. jr. et OSATO R.L. (JACS, (1951), 73, 5553), on obtient à partir d'acide myristique et de (S)-phénylalaninate de terbutyle le produit attendu.
Rendement : 92 %

**Stade B** : N-myristoyl-(S)-phénylalanine

2 g du composé obtenu au stade A sont dissous dans 25 ml de dichlorométhane. Après addition de 5 ml d'acide trifluoroacétique, la solution est abandonnée 24 heures à température ambiante. Elle est alors évaporée à sec. Le produit attendu est obtenu après cristallisation du résidu dans le pentane et filtration.

Rendement : 49 %
Point de fusion (Kofler) : 74°C

Microanalyse élémentaire :

|  | C % | H % | N % |
|---|---|---|---|
| Calculé : | 73,56 | 9,93 | 3,73 |
| Trouvé : | 73,30 | 9,81 | 3,58 |

**ETUDE PHARMACOLOGIQUE :**

**Exemple 2 : Méthode pour inhiber la N-Myristoyl Transférase (NMT) par des analogues du produit de la réaction qu'elle catalyse**

**Stade A** : Culture en masse de cellules cancéreuses L 1210

Des cellules leucémiques d'origine murine (L 1210) sont cultivées en roller de 500 à 1000 ml dans un milieu comprenant : milieu RPMI 1640 (Gibco) ; 10 % de sérum de veau foetal ; 50 unités/ml de pénicilline ; 50 $\mu$M de streptomycine ; 2 mM de glutamine et 10 mM de HEPES (Gibco). Les cellules croissent sous agitation permanente à 37°C dans une atmosphère de 5 % $CO_2$/95 % air. Les cellules sont recueillies et lavées. Le culot final, comportant typiquement 2,5 $10^{11}$ cellules est resuspendu dans un tampon de lyse [50 mM TRIS, pH 7,9 ; 1mM DTT ; 1mM EDTA et 250 mM de sucrose]. Les cellules sont lysées et homogénéisées. Les microsomes (réticulum endoplasmique vésiculisé) sont recueillis par centrifugation à 105 000 g comme culot.

**Stade B** : Solubilisation de l'enzyme à partir de cette source biologique

La NMT est une enzyme membranaire. Pour solubiliser cette enzyme, cette suspension est alors diluée au 2/3 dans un tampon de solubilisation : [50 mM HEPES, pH 7.5, 1 mM EDTA, 1 mM DTT, 10 % glycérol et 3 % de Triton 770] et maintenue pendant 30 min à 4°C sous agitation. Cette suspension est ensuite à nouveau centrifugée à 105 000 g pendant 75 min. Le matériel insoluble dans le détergent sédimente en culot. Le surnageant comprend la totalité de l'activité NMT présente dans les microsomes de L 1210.

**Stade C** : Mesure de l'activité N-Myristoyl Transférase et de son inhibition par la N-myristoyl-(S)-phénylalanine

Les composés sont alors testés sur le surnageant en compétition avec le substrat peptidique Gly-Asn-$(Ala)_4$-$(Arg)_2$ ("G8R") selon les conditions suivantes comme décrites par TOWLER et Coll. (PNAS, (1987), 84, 2708-2711). Le Myristoyl-Coenzyme A est synthétisé extemporanément et enzymatiquement par incubation de Myristate avec ATP, Coenzyme A (sel de lithium) et de l'Acyl-Coa synthetase de Pseudomonas (20 min à 30°C). Puis sont ajoutés : le peptide substrat et la source biologique telle qu'obtenue ci-dessus. Le mélange est incubé 10 min à 37°C ; puis la réaction est stoppée par 110 $\mu$l de méthanol et 10 $\mu$l d'acide trichloroacétique.

Le milieu est laissé à 4°C pendant 10 min, puis centrifugé à 10000 g pendant 5 min. Une aliquote (30 μl) du surnageant final est injecté dans un système HPLC équipé d'une colonne μBondapak® (Waters) et développé par un gradient linéaire tel que décrit par Towler et al (PNAS, (1987), 84, 2708-11). La détection du peptide acylé s'effectue à l'aide d'un détecteur radioactif en ligne (Berthold) grâce à l'adjonction de 5 ml/min de liquide de scintillation (Zinsser). Le peptide acylé est élué à 85 % d'acétonitrile-TFA/15 % H20-TFA.

Les composés testés comme inhibiteurs sont suspendus dans une solution à 0.1 % de Triton 770, après une brève sonication à froid. 10 μl de la solution d'inhibiteur sont rajoutés au milieu réactionel. Les conditions expérimentales sont identiques à celles décrites ci-dessus. L'activité du composé est évaluée sur la base de la diminution de l'activité contrôle, incubée dans les mêmes conditions, mais en présence de la solution de suspension.

Dans ces conditions, la N-myristoyl-(S)-phénylalanine présente une IC50 égale à $2.10^{-4}$ M. Ce qui correspond à une activité 15 fois supérieure à celle mesurée pour la N-myristoylglycine dont l'IC50 est égale à $3.10^{-3}$M.

## Exemple 3 : Cytotoxicité

La cytotoxicité des composés est appréciée par l'utilisation du test colorimétrique décrit par ALLEY et Coll. (Cancer Res., (1988), 48, 589-601). Ce test automatisé est utilisé en routine sur 2 lignées cellulaires: L 1210 (leucémie murine) et HL 60 (promyelocyte humain). Sur chacune de ces lignées, la cytotoxicité est appréciée en utilisant 9 concentrations des composés. L'IC50 est la concentration inhibant 50 % de la croissance des cellules.

Les résultats sont regroupés dans le tableau suivant :

|  | IC50 (M) sur cellules L 1210 | IC50 (M) sur cellules HL 60 |
|---|---|---|
| N-myristoyl-(S)-phénylalanine | 29,5 | 12,8 |
| N-myristoyl glycine | 48,4 | 20,5 |

Ces résultats montrent que la N-myristoyl-(S)-phénylalanine présente une cytotoxicité nettement supérieure à celle de la N-myristoyl glycine.

## COMPOSITION PHARMACEUTIQUE

### Exemple 4 : Comprimé : formule de préparation pour 1000 comprimés dosés à 2 mg de principe actif

```
N-myristoyl-(S)-phénylalanine  . . . . . . . . . . . .    2 g
Hydroxypropyl cellulose   . . . . . . . . . . . . . . .    2 g
Amidon de blé   . . . . . . . . . . . . . . . . . . . .   10 g
Lactose   . . . . . . . . . . . . . . . . . . . . . . .  100 g
Stéarate de magnésium   . . . . . . . . . . . . . . . .    3 g
Talc  . . . . . . . . . . . . . . . . . . . . . . . . .    3 g
```

**Revendications**

**Revendications pour les Etats contractants suivants : AT, BE, CH, DE, DK, FR, GB, IT, LI, LU, NL, SE**

1. Utilisation de la N-myristoyl-(S)-phénylalanine ou d'un de ses sels d'addition à une base pharmaceutiquement acceptable, pour l'obtention d'une composition pharmaceutique caractérisée par le fait qu'elle comprend un ou plusieurs excipients ou véhicules inertes, non toxiques, pharmaceutiquement acceptables, utilisable en tant qu'inhibiteur de N-myristoyl Transférase dans le traitement du cancer.

2. Composition pharmaceutique pour le traitement des maladies faisant intervenir la myristoylation et plus particulièrement le traitement du cancer contenant la N-myristoyl-(S)-phénylalanine ou un de ses sels d'addition à une base pharmaceutiquement acceptable.

**Revendications pour les Etats contractants suivants : ES, GR**

1. Utilisation de la N-myristoyl-(S)-phénylalanine ou d'un de ses sels d'addition à une base pharmaceutiquement acceptable, pour l'obtention d'une composition pharmaceutique caractérisée par le fait qu'elle comprend un ou plusieurs excipients ou véhicules inertes, non toxiques, pharmaceutiquement acceptables, utilisable en tant qu'inhibiteur de N-myristoyl Transférase dans le traitement du cancer.

2. Procédé pour préparer une composition pharmaceutique pour le traitement des maladies faisant intervenir la myristoylation et plus particulièrement le traitement du cancer par mélange de la N-myristoyl-(S)-phénylalaline ou un de ses sels d'addition à base pharmaceutiquement acceptable.

**Patentansprüche**

**Patentansprüche für folgende Vertragsstaaten : AT, BE, CH, DE, DK, FR, GB, IT, LI, LU, NL, SE**

1. Verwendung von N-Myristoyl-(S)-phenylalanin oder eines Säureadditionssalzes davon mit einer pharmazeutisch annehmbaren Base zur Herstellung einer pharmazeutischen Zubereitung, **dadurch gekennzeichnet** ist, daß sie eine oder mehrere inerte, nichttoxische, pharmazeutisch annehmbare Trägermaterialien oder Hilfsstoffe enthält und als Inhibitor der N-Myristoyltransferase bei der Behandlung von Krebs verwendet werden kann.

2. Pharmazeutische Zubereitung zur Behandlung von Erkrankungen, bei denen eine Myristoylierung erfolgt, und insbesondere zur Behandlung von Krebs, enthaltend N-Myristoyl-(S)-phenylalanin oder eines seiner Additionssalze mit einer pharmazeutisch annehmbaren Base.

**Patentansprüche für folgende Vertragsstaaten : ES, GR**

1. Verwendung von N-Myristoyl-(S)-phenylalanin oder eines Säureadditionssalzes davon mit einer pharmazeutisch annehmbaren Base zur Herstellung einer pharmazeutischen Zubereitung, **dadurch gekennzeichnet** ist, daß sie eine oder mehrere inerte, nichttoxische, pharmazeutisch annehmbare Trägermaterialien oder Hilfsstoffe enthält und als Inhibitor der N-Myristoyltransferase bei der Behandlung von Krebs verwendet werden kann.

2. Verfahren zur Herstellung einer pharmazeutischen Zubereitung zur Behandlung von Erkrankungen, bei denen eine Myristoylierung erfolgt, und insbesondere zur Behandlung von Krebs, durch Vermischen von N-Myristoyl-(S)-phenylalanin oder eines seiner Additionssalze mit einer pharmazeutisch annehmbaren Base.

**Claims**

**Claims for the following Contracting States : AT, BE, CH, DE, DK, FR, GB, IT, LI, LU, NL, SE**

1. Use of N-myristoyl-(S)-phenylalanine, or an addition salt thereof with a pharmaceutically acceptable base, for obtaining a pharmaceutical composition that is characterised in that it comprises one or more inert, non-toxic, pharmaceutically acceptable excipients or carriers and that can be used as an inhibitor of N-myristoyl transferase in the treatment of cancer.

2. Pharmaceutical composition for the treatment of disorders involving myristoylation, and more especially the treatment of cancer, containing N-myristoyl-(S)-phenylalanine or an addition salt thereof with a pharmaceutically acceptable base.

**Claims for the following Contracting States : ES, GR**

1. Use of N-myristoyl-(S)-phenylalanine, or an addition salt thereof with a pharmaceutically acceptable base, for obtaining a pharmaceutical composition that is characterised in that it comprises one or more inert, non-toxic, pharmaceutically acceptable excipients or carriers and that can be used as an inhibitor of N-myristoyl transferase in the treatment of cancer.

2. Process for the preparation of a pharmaceutical composition for the treatment of disorders involving myristoylation, and more especially the treatment of cancer, by mixing N-myristoyl-(S)-phenylalanine or an addition salt thereof with a pharmaceutically acceptable base.